# EUROPEAN PATENT SPECIFICATION

(11) **EP 3 919 010 B1**
(45) Date of publication and mention of the grant of the patent: **11.12.2024**
(21) Application number: 21177618.2
(22) Date of filing: 03.06.2021
(51) Int. Cl.: A61B 17/34, A61B 17/29

(54) **SURGICAL ACCESS DEVICE INCLUDING ADJUSTABLE CANNULA PORTION**
CHIRURGISCHE ZUGANGSVORRICHTUNG MIT VERSTELLBAREM KANÜLENABSCHNITT
DISPOSITIF D'ACCÈS CHIRURGICAL DOTÉ D'UNE PARTIE DE CANULE RÉGLABLE

(30) Priority: 04.06.2020 US 202063034698 P; 14.05.2021 US 202117320370
(43) Date of publication of application: 08.12.2021
(73) Proprietor: Covidien LP, Mansfield, MA 02048 (US)
(72) Inventor: GEORGE, Sabastian K., 562125 Bangalore (IN)
(74) Representative: Maschio & Soames IP Ltd

(56) References cited:
- EP-A2- 3 420 985
- US-A1- 2011 144 442
- US-A1- 2011 144 444
- US-A1- 2013 053 777
- US-A1- 2013 245 370
- US-A1- 2015 141 938

## Description

### CROSS-REFERENCE TO RELATED APPLICATION

This application claims priority to, and the benefit of, U.S. Provisional Patent Application Serial No. 63/034,698 filed on June 4, 2020.

### TECHNICAL FIELD

The present disclosure relates to a surgical access device. More particularly, the present disclosure relates to surgical access devices having adjustable cannula portions.

### BACKGROUND OF RELATED ART

Endoscopic and laparoscopic minimally invasive procedures have been used for introducing medical devices inside a patient and for viewing portions of the patient's anatomy. To operate on a desired anatomical site, a surgeon may insert a rigid or flexible endoscope inside the patient to physically engage with the anatomical site.

Typically, a trocar assembly includes a cannula and an obturator. The cannula remains in place for use during the laparoscopic procedure, and the obturator includes a tip for penetrating body tissue. In endoscopic surgical procedures, surgery is performed in any hollow organ or tissue of the body through a small incision or through a narrow endoscopic tube (e.g., a cannula) inserted through a small entrance wound in the skin. In laparoscopic procedures, surgical operations in the abdomen are performed through small incisions (usually about 0.5 to about 1.5 cm). Laparoscopic and endoscopic procedures often require the surgeon to act on organs, tissues, and vessels at varying distances from the incision. Examples of prior art cannulas that are movable between different configurations are disclosed in US 2011/144444 A1, US 2015/141938 A1, EP 3 420 985 A2, US 2011/144442 A1 and US 2013/053777 A1.

Accordingly, it may be helpful to provide an adjustable trocar assembly that is configured to provide flexibility of depth within a surgical site.

### SUMMARY

The present invention provides an adjustable trocar assembly as claimed in claim 1 appended hereto, the assembly including a housing configured to facilitate insertion of one or more surgical tools into a patient's body and an elongated tubular member extending distally from the housing and defining a longitudinal axis. Further embodiments are defined by the dependent claims. The elongated tubular member includes a channel configured to facilitate passage of surgical instruments through the elongated tubular member, an adjustable portion configured to facilitate axial movement of the elongated tubular member, a collar of fixed diameter, and a rim of fixed diameter equal to the diameter of the collar.

The adjustable portion is defined by a plurality of adjacent circular ridges centered along the longitudinal axis of the elongated tubular member.

Each circular ridge of the plurality of circular ridges is operably coupled to one or more adjacent circular ridges of the plurality of circular ridges by a flexible material interconnecting adjacent circular ridges.

The collar may be positioned along the elongated tubular member where the channel transitions into the adjustable portion and the rim is disposed on the distal end of the adjustable portion.

A small portion of excess material defines a fold in each circular ridge of the plurality of circular ridges. The fold is configured to collapse inward and facilitate partial insertion of one circular ridge of the plurality of circular ridges into a proximally adjacent circular ridge of the plurality of circular ridges.

The proximal end of each circular ridge of the plurality of circular ridges may be configured to be inserted into the distal end of the proximally adjacent circular ridge of the plurality of circular ridges, and the distal end of each circular ridge of the plurality of circular ridges may be configured to receive the proximal end of the distally adjacent circular ridge of the plurality of circular ridges when in a retracted state.

The distal end of each circular ridge of the plurality of circular ridges may be configured to be separated from the proximal end of the distally adjacent circular ridge of the plurality of circular ridges by a length of a fold when in an extended state.

The collar and the rim may be made from a rigid material.

Each pair of adj acent circular ridges may interact independently from all other non-adjacent circular ridges, such that the adjustable portion can be partially extended or partially retracted.

Adjacent circular ridges of the plurality of circular ridges are interconnected by a layer of thin and flexible material.

Falling outside the scope of the present invention but disclosed herein is a method of using an adjustable trocar assembly. The method includes introducing one or more surgical tools into a channel defined by an elongated tubular member, wherein the elongated tubular member includes a plurality of adjacent circular ridges coupled to one another by a flexible material defining an adjustable portion, and wherein a collar of fixed diameter and a rim of fixed diameter cooperate to ensure that axial uniformity is maintained along the adjustable portion of the elongated tubular member. The method further includes moving the one or more surgical tools through the channel and the adjustable portion until the distal ends of the one or more surgical tools engage the rim of the adjustable portion.

The method may further include extending the adjustable portion toward a target site in a patient's body by exerting a force on the rim in the distal direction until the separation between the distal ends of at least one pair of adj acent of circular ridges, of the plurality of circular ridges, is increased. Additionally, the method includes retracting the adjustable portion away from the target site in the patient's body by exerting a force on the rim in the proximal direction, until at the proximal end of at least one circular ridge of the plurality of circular ridges is partially inserted into the distal end of an adjacent circular ridge of the plurality of circular ridges.

Other features of the disclosure will be appreciated from the following description.

### BRIEF DESCRIPTION OF THE DRAWINGS

Various aspects of the present disclosure are illustrated herein with reference to the accompanying drawings, wherein:
FIG. 1 is a perspective view of an adjustable trocar assembly including a cannula and an obturator;
FIG. 2A is a perspective view of an aspect of an adjustable portion of the adjustable trocar assembly of FIG. 1 in a compressed state;
FIG. 2B is a perspective view of an aspect of an adjustable portion of the adjustable trocar assembly of FIG. 1 in an unaltered state;
FIG. 2C is a perspective view of an aspect of an adjustable portion of the adjustable trocar assembly of FIG. 1 in an extended state;
FIG. 3A is a cross-sectional view along line A-A of the adjustable portion of FIG. 2A;
FIG. 3B is a cross-sectional view along line B-B of the adjustable portion of FIG. 2B;
FIG. 3C is a cross-sectional view along line C-C of the adjustable portion of FIG. 2C;
FIG. 3D is a cross-sectional view along line D-D of the adjustable portion of FIG. 2A;
FIG. 4A is a side view of the trocar assembly of FIG. 1 engaged with tissue in a fully compressed state;
FIG. 4B is a side view of the trocar assembly of FIG. 1 engaged with tissue in a partially extended state; and
FIG. 4C is a side view of the trocar assembly of FIG. 1 engaged with tissue in a fully extended state.

### DETAILED DESCRIPTION

Aspects of the presently disclosed surgical access device with an adjustable cannula portion are described in detail with reference to the drawings, wherein like reference numerals designate corresponding elements in each of the several views.

As used herein, the term "distal" refers to that portion of the instrument, or component thereof which is farther from the user while the term "proximal" refers to that portion of the instrument or component thereof which is closer to the user.

Various aspects of a surgical access device are described herein. Generally, the surgical access device includes a trocar assembly which may be employed during surgery (e.g., laparoscopic surgery) and may, in various aspects, provide for the sealed access of laparoscopic surgical instruments into an insufflated body cavity, such as the abdominal cavity. As will be described in additional detail below, the step of the present disclosure includes a cannula and an obturator insertable therethrough. The cannula and obturator are separate components but are capable of being selectively connected together. For example, the obturator may be inserted into and through the cannula until the handle of the obturator engages, e.g., selectively locks into, a proximal housing of the cannula. In this initial position, the trocar assembly is employed to tunnel through an anatomical structure, e.g., the abdominal wall, either by making a new passage through the structure or by passing through an existing opening through the structure. Once the trocar assembly has tunneled through the anatomical structure, the obturator is removed, leaving the cannula in place in the structure, e.g., in the incision created by the trocar assembly. The proximal housing of the cannula may include seals or valves that prevent the escape of insufflation gases from the body cavity, while also allowing surgical instruments to be inserted into the body cavity. Further details of a surgical access device including a cannula and an obturator are described in U.S. Patent No. 10,022,149 to Holsten et al., issued on July 17, 2018, and U.S. Patent Application Publication No. 2018/0085145 to Okoniewski et al., filed on November 13, 2017.

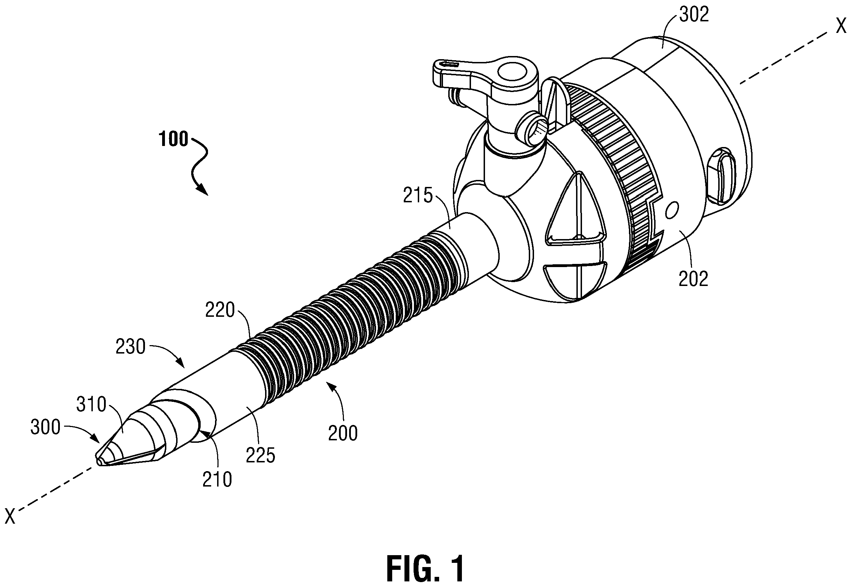

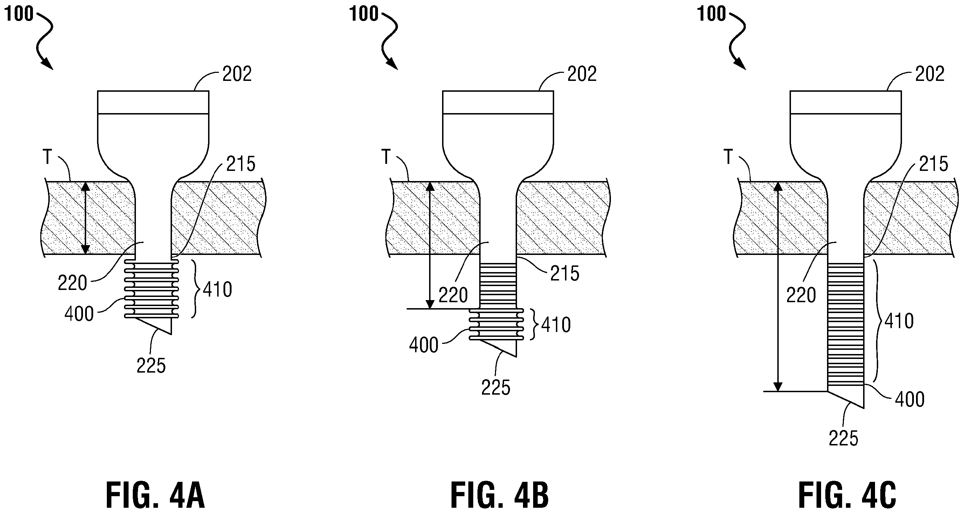

With initial reference to FIG. 1, an adjustable trocar assembly 100 is shown. The adjustable trocar assembly 100 includes a cannula 200, and an obturator 300. The obturator 300 is insertable through a channel 210 defined by an elongated tubular member 220 of cannula 200, which defines a longitudinal axis X-X. Additionally, obturator 300 is selectively engageable with or attachable to cannula 200. More particularly, a proximal portion 302 of obturator 300 is selectively engageable with or attachable to a proximal portion or housing 202 of cannula 200. In use, when obturator 300 is engaged with cannula 200, a distal end 310 of obturator 300 is advanced into tissue "T" to create or enlarge an incision or opening in tissue "T" (FIGS. 4A-C). Alternatively, a distal end 230 of cannula 200 can be used to create or enlarge an opening in tissue "T," without the use of an obturator, for instance. In either situation, it may be important to limit or control the insertion depth of cannula 200 with respect to the tissue "T" to help provide optimal access to the target tissue, and to minimize accidental contact between portions of cannula 200 or obturator 300 with tissue located distally of the target tissue. The adjustable portion 400 of the present disclosure is positionable on cannula 200, and is configured to provide a wider range of motion in which cannula 200 can be extended or retracted with respect to the tissue "T."

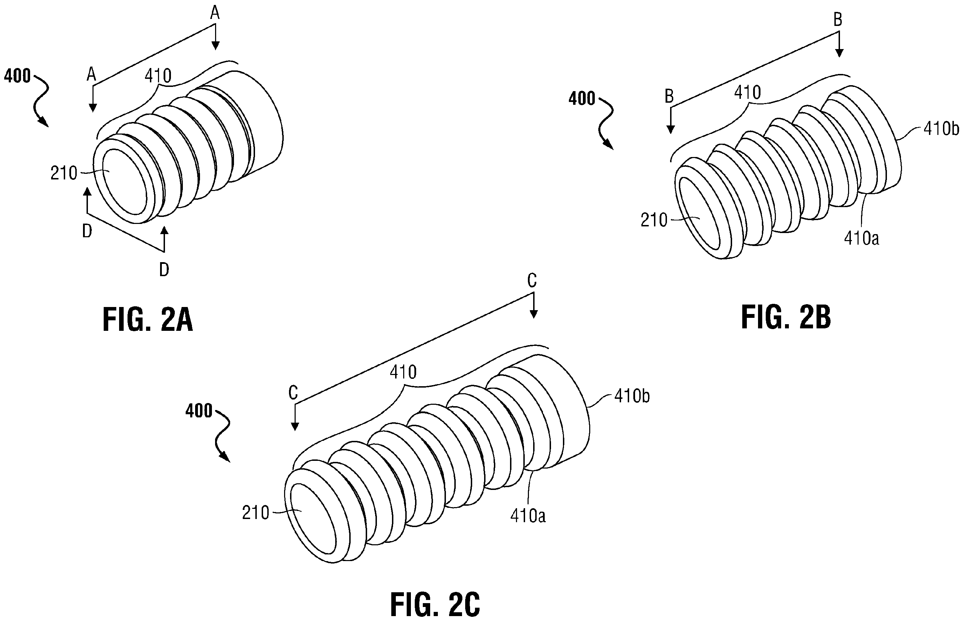

With particular reference to FIGS. 2A-3D, several views of the adjustable portion 400 are shown, which illustrate how the adjustable portion 400 has been configured to facilitate axial extension or axial retraction of the elongated tubular member 220. More specifically, the adjustable portion is comprised of a plurality of adjacent concentric circular ridges 410 centered along the longitudinal axis X-X of the elongated tubular member 220. Adj acent concentric circular ridges 410 of elongated tubular member 220 are interconnected by a thin layer of flexible material that defines an internal surface 420 and an external surface 430.

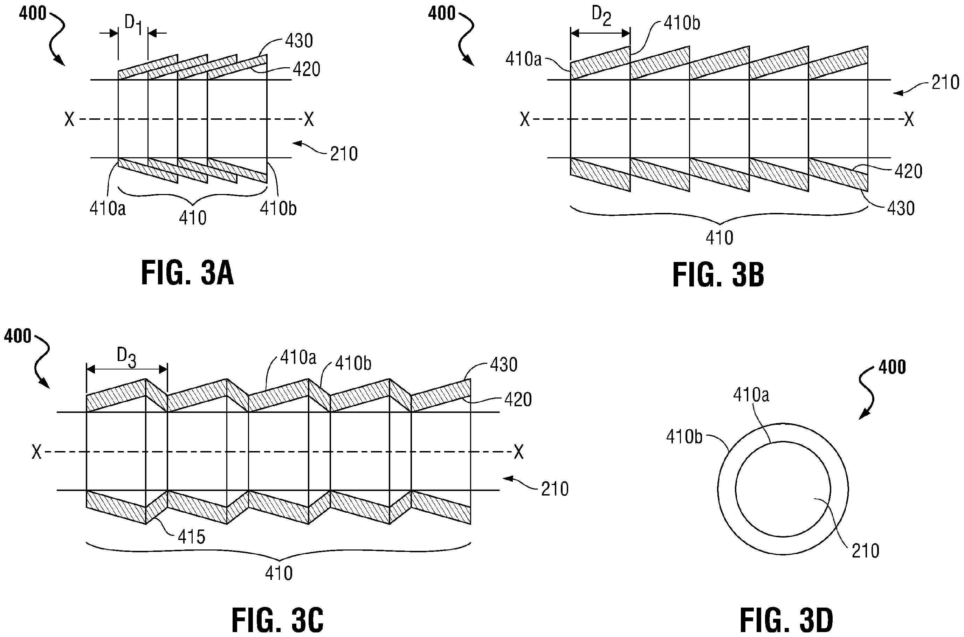

Each of the plurality of circular ridges 410 includes a proximal end 410a and a distal end 410b such that the radius of the proximal end 410a is less than the radius of the radius of the distal end 410b, as can be seen in FIG. 3D. The difference in radius corresponds to a difference in circumference between the proximal end 410a and the distal end 410b, and creates a tapering effect along the external surface 430 between adjacent ridges 400, as can be seen in FIGS 3A-3C. Within channel 210 of the elongated tubular member, the tapering effect of each of the plurality of circular ridges 410 allows for the proximal end 410a to be partially inserted into the proximally adjacent circular ridge 410, such that the minimum axial radius of the internal surface 420 never decreases below the radius of the proximal end 410a. As such, the tapering effect ensures that a minimum axial radius exists along the internal surface 420 of the adjustable portion 400 regardless of whether adjustable portion 400 is retracted, extended, or unaltered, as can be seen in FIGS 3A-3C. The minimum axial radius along the internal surface 420 of the adjustable portion 400 ensures that there is axial uniformity throughout the entire length of the elongated tubular member 220 including adjustable portion 400, i.e., adjustable portion 400 has a uniform inner and outer diameter. Correspondingly, this axial uniformity along internal surface 420 allows for any standard sized obturator 300 to be inserted through channel 210 regardless of whether the adjustable portion 400 is retracted, extended, or unaltered.

In addition to the proximal end 410a and the distal end 410b, each circular ridge of the plurality of circular ridges 410 includes a fold 415 made of a small portion of excess material from the thin and flexible material used to interconnect adjacent circular ridges 410. In order to facilitate the partial insertion of one of the ridges 410 into the proximally adjacent ridge 410, the internal surface 420 of the fold 415 is configured to fold/collapse in on itself between the adjacent circular ridges 410 when the adjustable portion 400 is compressed/retracted, as shown in FIGS. 2A and 3A. Conversely, when adjustable portion 400 is fully extended, as shown in FIGS. 2C and 3C, the fold 415 is configured to unfold and to extend distally outward to increase the separation between adjacent circular ridges 410.

When adjustable portion 400 is compressed/fully retracted, as shown in FIGS. 2A and 3A, the tapering effect allows for each proximal end 410a to pass through the distal end 410b and be partially inserted into the adjacent circular ridge 410. Measurement of a distance D1 between the proximal ends 410a of adjacent circular ridges 410 when the adjustable portion 400 is compressed/retracted shows where the distance D1 is less than a similarly measured distance D2 between the proximal ends 410a of adjacent circular ridges 410 when the adjustable portion 400 is in an unaltered state, as seen in FIGS. 2B and 3B. Further, both distances D1 and D2 are shown to be less than a distance D3 measured between the proximal ends 410a of adjacent circular ridges 410 when the adjustable portion 400 is fully extended, as shown in FIGS. 2C and 3C.

Now referring to FIGS. 4A-C, in use the adjustable trocar assembly 100 is inserted through a patient's abdominal wall to provide the obturator 300 with access to area being targeted for treatment. Depending on where the area being targeted is located within the patient's body, obturator 300 may need to be inserted farther into the body cavity. Correspondingly, the length of obturator 300 being used will also vary according to this need and the length of the channel 210 through which obturator 300 is inserted will also need to be variable. Traditionally, when a need arises to operate on a target area deeper than expected, the surgeon would need to remove the cannula in use and replace it with a longer cannula. This process of exchanging one cannula for another can be time consuming and introduces an additional risk of complication to the procedure. Adjustable portion 400 provides benefit of variable length without a need to exchange one cannula for another, and without adding any risk of complications to the procedure. Instead of removing one piece of equipment from the patient's body and inserting another, the adjustable portion 400 of the elongated tubular member 220 can simply be extended as needed. Additionally, the variable length of adjustable portion 400 can be easily adjusted to match the thickness of a patient's tissue layer(s).

FIG. 1 shows another aspect of the adjustable trocar assembly 100, where the elongated tubular member 220 further includes a collar 215 positioned along the elongated tubular member 220 at the point where the channel 210 transitions into the adjustable portion 400 of elongated tubular member 220, and a rim 225 positioned at the distal end of the elongated member 220. Together the collar 215 and rim 225 cooperate to ensure that axial uniformity is maintained along the elongated tubular member 220. Unlike the retractable and extendable circular ridges 410 positioned in between the collar 215 and the rim 225, the collar 215 and rim 225 are rigid structures that cannot extend or retract. Instead, both the collar 215 and the rim 225 define rings of equal radii configured to ensure that a minimum axial radius throughout the length of adjustable portion 400.

From the earlier discussion of the tapering effect between adjacent circular ridges 410, as shown in FIGS. 2A-3D, it will be appreciated that each of the plurality of circular ridges 410 is configured to be partially inserted into the distal end 410b of the proximally adjacent circular ridge 410, and that the distal end 410b of each of the plurality of circular ridges 410 is configured to receive the proximal end 410a of the distally adjacent circular ridge 410 when in the retracted state. The rigidity of collar 215 allows the collar 215 to act as a brace that can receive the proximal end 410a of the proximal-most circular ridge 410 of the adjustable portion 400. Once inserted into the collar 215 the second most proximal circular ridge 410 can then receive the proximal end 410a of the third most proximal circular ridge 410 and so on until eventually all of the adjacent circular ridges 410 have been partially inserted and the adjustable portion 400 is in a fully retracted state.

Given the thinness and flexibility of the material used to interconnect the plurality of circular ridges 410, the tapering effect between adjacent circular ridges 410 requires an initial rigid form to support the first partial insertion of one ridge 410 into another. The collar 215 provides that initial rigid support structure that is relied upon by all subsequent adjacent circular ridges 410. As such, the collar 215 defines the minimum axial radius and by extension ensures axial uniformity along the elongated tubular member 220. Similarly, the rim 225 disposed on the distal end of elongated member 220 acts as a brace for the final circular ridge 410 and provides a well-defined stopping point that can independently maintain its shape and rigid form. The rigidity of rim 225 ensures that the distal-most circular ridge 410 maintains its shape and as such also ensures axial uniformity along the elongated tubular member 220. Additionally, the rim 225 acts as a guide for any surgical instruments inserted therethrough by ensuring that upon exiting the rim said surgical instruments are facing in the direction of the area being targeted for treatment.

In other aspects of the adjustable portion 400, a semi-rigid material can also be used to interconnect the plurality of circular ridges 410, such that each pair of adjacent circular ridges 410 can interact completely independently of all other non-adjacent circular ridges 410 comprising the adjustable portion 400. This independence can facilitate partial extension and retraction of the adjustable portion 400, regardless of the rigidity of the collar 215.

While the above description contains many specifics, these specifics should not be construed as limitations on the scope of the present disclosure, but merely as illustrations of various aspects thereof. Therefore, the above description should not be construed as limiting, but merely as exemplifications of various aspects.

## Claims

1. An adjustable trocar assembly (100) comprising:
a housing (202) configured to facilitate insertion of one or more surgical tools into a patient's body; and
an elongated tubular member (220) extending distally from the housing and defining a longitudinal axis, the elongated tubular member including:
a channel (210) configured to facilitate passage of surgical instruments through the elongated tubular member;
an adjustable portion (400) configured to facilitate axial extension or axial retraction of the elongated tubular member, wherein the adjustable portion is defined by a plurality of adjacent extendable and retractable circular ridges (410) centered along the longitudinal axis of the elongated tubular member, each of the circular ridges (410) including a proximal end (410a) and a distal end (410b), wherein the radius of the proximal end is less than the radius of the distal end to create a tapering effect along an external surface (430) between adjacent ridges to allow for the proximal end (410a) to be partially inserted into the proximally adjacent circular ridge (410) so that the minimum axial radius of the internal surface (420) of the tubular member never decreases below the radius of the proximal end (410a) of each circular ridge (410);
a collar (215) of fixed diameter; and
a rim (225) of fixed diameter equal to the diameter of the collar; **characterized by** each of the circular ridges (410) of the adjustable portion being operably coupled to one or more adjacent circular ridges of the plurality of circular ridges by a flexible material interconnecting adjacent circular ridges wherein each circular ridge includes a fold (415) made by an excess of a small portion of the flexible material, the fold configured to collapse inward between adjacent circular ridges to facilitate partial insertion of one circular ridge into an adjacent circular ridge during retraction of the adjustable portion (400).

2. The adjustable trocar assembly of claim 1, wherein the collar (215) is positioned along the elongated tubular member (220) where the channel (210) transitions into the adjustable portion (400) and the rim (225) is disposed on the distal end of the adjustable portion.

3. The adjustable trocar assembly of any one of the preceding claims wherein the proximal end (410a) of each circular ridge (410) of the plurality of circular ridges is configured to be inserted into the distal end (410b) of the proximally adjacent circular ridge of the plurality of circular ridges and the distal end (410b) of each circular ridge of the plurality of circular ridges is configured to receive the proximal end (410a) of the distally adjacent circular ridge of the plurality of circular ridges when in a retracted state.

4. The adjustable trocar assembly of claim 3, wherein the distal end (410b) of each circular ridge (410) of the plurality of circular ridges is configured to be separated from the proximal end (410a) of the distally adjacent circular ridge of the plurality of circular ridges by a length of the fold (415) when in an extended state.

5. The adjustable trocar assembly of claim 2, wherein the collar (215) and rim (225) are made from a rigid material.

6. The adjustable trocar assembly of claim 5, wherein each pair of adjacent circular ridges (410) are interconnected by a semi-rigid material such that each pair is able to interact independently from all other non-adjacent circular ridges, such that the adjustable portion can be partially extended or partially retracted.

## Patentansprüche

1. Einstellbare Trokaranordnung (100), umfassend:
ein Gehäuse (202), das so konfiguriert ist, dass es das Einführen eines oder mehrerer chirurgischer Werkzeuge in den Körper eines Patienten erleichtert; und
ein langgestrecktes röhrenförmiges Element (220), das sich distal von dem Gehäuse erstreckt und eine Längsachse definiert, wobei das langgestreckte röhrenförmige Element einschließt:
einen Kanal (210), der so konfiguriert ist, dass er den Durchgang von chirurgischen Instrumenten durch das langgestreckte röhrenförmige Element erleichtert;
einen einstellbaren Abschnitt (400), der so konfiguriert ist, dass er die axiale Ausdehnung oder das axiale Zurückziehen des langgestreckten röhrenförmigen Elements erleichtert, wobei der einstellbare Abschnitt durch eine Vielzahl benachbarter ausfahrbarer und zurückziehbarer kreisförmiger Stege (410) definiert ist, die entlang der Längsachse des langgestreckten röhrenförmigen Elements zentriert sind, wobei jeder der kreisförmigen Stege (410) ein proximales Ende (41 0a) und ein distales Ende (41 0b) einschließt, wobei der Radius des proximalen Endes kleiner ist als der Radius des distalen Endes, um einen Verjüngungseffekt entlang einer Außenfläche (430) zwischen benachbarten Rippen zu erzeugen, um zu ermöglichen, dass das proximale Ende (410a) teilweise in den proximal benachbarten kreisförmigen Steg (410) eingeführt wird, so dass der minimale axiale Radius der Innenfläche (420) des rohrförmigen Elements niemals unter den Radius des proximalen Endes (410a) jedes kreisförmigen Stegs (410) abnimmt;
einen Kragen (215) mit festem Durchmesser; und
einen Rand (225) mit festem Durchmesser, der gleich dem Durchmesser des Kragens ist; **dadurch gekennzeichnet, dass** jeder der kreisförmigen Stege (410) des einstellbaren Abschnitts mit einem oder mehreren benachbarten kreisförmigen Stegen der Vielzahl von kreisförmigen Stegen durch ein flexibles Material, das benachbarte kreisförmige Stege miteinander verbindet, funktionsfähig gekoppelt ist, wobei jeder kreisförmige Steg eine Falte (415) aufweist, die durch einen Überschuss eines kleinen Abschnitts des flexiblen Materials gebildet wird, wobei die Falte so konfiguriert ist, dass sie zwischen benachbarten kreisförmigen Stegen nach innen kollabiert, um ein teilweises Einführen eines kreisförmigen Stegs in einen benachbarten kreisförmigen Steg während des Zurückziehens des einstellbaren Abschnitts (400) zu erleichtern.

2. Einstellbare Trokaranordnung nach Anspruch 1, wobei der Kragen (215) entlang des länglichen röhrenförmigen Elements (220) positioniert ist, wo der Kanal (210) in den einstellbaren Abschnitt (400) übergeht, und der Rand (225) an dem distalen Ende des einstellbaren Abschnitts angeordnet ist.

3. Einstellbare Trokaranordnung nach einem der vorhergehenden Ansprüche, wobei das proximale Ende (410a) jedes kreisförmigen Steges (410) der Vielzahl von kreisförmigen Stegen so konfiguriert ist, dass es in das distale Ende (410b) des proximal benachbarten kreisförmigen Steges der Vielzahl von kreisförmigen Stegen eingeführt wird, und das distale Ende (410b) jedes kreisförmigen Steges der Vielzahl von kreisförmigen Stegen so konfiguriert ist, dass es das proximale Ende (410a) des distal benachbarten kreisförmigen Steges der Vielzahl von kreisförmigen Stegen aufnimmt, wenn es sich in einem zurückgezogenen Zustand befindet.

4. Einstellbare Trokaranordnung nach Anspruch 3, wobei das distale Ende (410b) jedes kreisförmigen Steges (410) der Vielzahl von kreisförmigen Stegen so konfiguriert ist, dass es von dem proximalen Ende (410a) des distal benachbarten kreisförmigen Steges der Vielzahl von kreisförmigen Stegen durch eine Länge der Falte (415) getrennt ist, wenn es sich in einem ausgefahrenen Zustand befindet.

5. Einstellbare Trokaranordnung nach Anspruch 2, wobei der Kragen (215) und der Rand (225) aus einem starren Material hergestellt sind.

6. Einstellbare Trokaranordnung nach Anspruch 5, wobei jedes Paar benachbarter kreisförmiger Stege (410) durch ein halbstarres Material miteinander verbunden ist, so dass jedes Paar in der Lage ist, unabhängig von allen anderen nicht benachbarten kreisförmigen Stegen zu interagieren, so dass der einstellbare Abschnitt teilweise ausgefahren oder teilweise eingezogen werden kann.

## Revendications

1. Ensemble trocart ajustable (100) comprenant :
un logement (202) conçu pour faciliter l'insertion d'un ou plusieurs outils chirurgicaux dans le corps d'un patient ; et
un élément tubulaire allongé (220) s'étendant distalement à partir du logement et définissant un axe longitudinal, l'élément tubulaire allongé comportant :
un canal (210) conçu pour faciliter le passage des instruments chirurgicaux à travers l'élément tubulaire allongé ;
une partie ajustable (400) conçue pour faciliter l'extension axiale ou la rétraction axiale de l'élément tubulaire allongé, dans lequel la partie ajustable est définie par une pluralité d'arêtes circulaires (410) adjacentes extensibles et rétractables centrées le long de l'axe longitudinal de l'élément tubulaire allongé, chacune des arêtes circulaires (410) comportant une extrémité proximale (410a) et une extrémité distale (410b), dans lequel le rayon de l'extrémité proximale est inférieur au rayon de l'extrémité distale afin de créer un effet d'amincissement le long d'une surface externe (430) entre les arêtes adjacentes pour permettre à l'extrémité proximale (410a) d'être partiellement insérée dans l'arête circulaire (410) proximalement adjacente de sorte que le rayon axial minimum de la surface interne (420) de l'élément tubulaire ne diminue jamais en dessous du rayon de l'extrémité proximale (410a) de chaque arête circulaire (410) ;
un collier (215) de diamètre fixe ; et
un rebord (225) de diamètre fixe égal au diamètre du collier ; **caractérisé par le fait que** chacune des arêtes circulaires (410) de la partie ajustable est accouplée de manière opérationnelle à une ou plusieurs arêtes circulaires adjacentes de la pluralité d'arêtes circulaires par un matériau flexible reliant les arêtes circulaires adjacentes entre elles, dans lequel chaque arête circulaire comporte un pli (415) fait par un excès d'une petite partie du matériau flexible, le pli étant conçu pour s'effondrer vers l'intérieur entre les arêtes circulaires adjacentes afin de faciliter l'insertion partielle d'une arête circulaire dans une arête circulaire adjacente pendant la rétraction de la partie ajustable (400).

2. Ensemble trocart ajustable selon la revendication 1, dans lequel le collier (215) est positionné le long de l'élément tubulaire allongé (220) où le canal (210) passe dans la partie ajustable (400) et le rebord (225) est disposé sur l'extrémité distale de la partie ajustable.

3. Ensemble trocart ajustable selon l'une quelconque des revendications précédentes, dans lequel l'extrémité proximale (410a) de chaque arête circulaire (410) de la pluralité d'arêtes circulaires est conçue pour être insérée dans l'extrémité distale (410b) de l'arête circulaire proximalement adjacente de la pluralité d'arêtes circulaires et l'extrémité distale (410b) de chaque arête circulaire de la pluralité d'arêtes circulaires est conçue pour recevoir l'extrémité proximale (410a) de l'arête circulaire distalement adjacente de la pluralité d'arêtes circulaires dans un état rétracté.

4. Ensemble trocart ajustable selon la revendication 3, dans lequel l'extrémité distale (410b) de chaque arête circulaire (410) de la pluralité d'arêtes circulaires est conçue pour être séparée de l'extrémité proximale (410a) de l'arête circulaire distalement adjacente de la pluralité d'arêtes circulaires par une longueur du pli (415) dans un état étendu.

5. Ensemble trocart ajustable selon la revendication 2, dans lequel le collier (215) et le rebord (225) sont fabriqués à partir d'un matériau rigide.

6. Ensemble trocart ajustable selon la revendication 5, dans lequel chaque paire d'arêtes circulaires adjacentes (410) sont reliées entre elles par un matériau semi-rigide de telle sorte que chaque paire peut interagir indépendamment de toutes les autres arêtes circulaires non adjacentes, de telle sorte que la partie ajustable peut être partiellement étendue ou partiellement rétractée.
